# EUROPÄISCHE PATENTSCHRIFT

(11) **EP 4 174 467 B1**
(45) Veröffentlichungstag und Bekanntmachung des Hinweises auf die Patenterteilung: **02.07.2025**
(21) Anmeldenummer: 22200264.4
(22) Anmeldetag: 07.10.2022
(51) Int. Cl.: G01L 19/00, G01L 19/04, G01L 9/00, A61M 5/168, A61B 5/021

(54) **GELGEKOPPELTE DRUCKSENSOREINRICHTUNG MIT STÖREINFLUSSUNABHÄNGIGER KONTAKTSEITE ZUR ANBINDUNG AN EINEN INFUSIONSSCHLAUCH**
GEL-COUPLED PRESSURE SENSOR DEVICE WITH DISTURBANCE-INDEPENDENT CONTACT SIDE FOR CONNECTION TO AN INFUSION TUBE
DISPOSITIF DE CAPTEUR DE PRESSION COUPLÉ À UN GEL AVEC UNE FACE DE CONTACT INDÉPENDANTE DE L'INFLUENCE DU BRUIT POUR LA CONNEXION À UN TUBE DE PERFUSION

(30) Priorität: 29.10.2021 DE 102021128378
(43) Veröffentlichungstag der Anmeldung: 03.05.2023
(73) Patentinhaber: B. Braun Melsungen AG, 34212 Melsungen (DE)
(72) Erfinder: BRESSAU, Ernst, 34323 Malsfeld (DE); KAUBA, Michael, 34277 Fuldabrück (DE); MORITZEN, Hans-Christian, 34119 Kassel (DE)
(74) Vertreter: Winter, Brandl - Partnerschaft mbB

(56) Entgegenhaltungen:
- DE-A1- 2 647 402
- DE-C1- 19 610 828
- DE-U1- 29 602 065

## Beschreibung

### Technisches Gebiet

Die vorliegende Offenbarung betrifft eine gelgekoppelte Drucksensoreinrichtung gemäß dem Oberbegriff des Patentanspruch 1 und insbesondere eine gelgekoppelte Drucksensoreinrichtung, die an (flexible) Schläuche, insbesondere medizinische Infusionsschläuche, zur im Wesentlichen messschwankungsfreien Schlauchinnendruckerfassung montiert werden kann.

### Hintergrund der Erfindung

In der Medizintechnik werden flexible Schläuche verwendet, um beispielsweise durch Infusion einem Patienten beispielweise Flüssigkeit zuzuführen, abzunehmen oder um Flüssigkeit zwischen Geräten bzw. Vorrichtungen zu befördern. Derartige Schläuche werden aber auch beispielsweise für Blutbehandlungsmaschinen, Herz-Lungen-Maschinen und dergleichen intensivmedizinische Geräte verwendet. Dabei besteht die Notwendigkeit, einen teilweisen oder völligen Verschluss des flexiblen Schlauches, wie er beispielsweise durch Abknicken des Schlauches auftritt, zu erkennen. Zudem ist es notwendig, je nach eingestellten Infusionsparametern den Innendruck des Infusionsschlauchs zu überwachen und entsprechend den eingestellten Parameter zu regulieren.

### Stand der Technik

Aus der EP 1 269 145 B1, EP 0 897 528 B1, EP 0 594 836 B1,EP 1 357 372 B1 sowie der DE 196 10 828 C1 und der DE 296 02 065 U1 sind jeweils Drucksensoreinrichtungen bekannt, in der Regel bestehend aus unter anderem einem Drucksensorgehäuse, in welchem ein Druckaufnahme- und Druckübertragungsmedium bzw. Druckübertragungselement angeordnet ist und einem Druckkraftsensor, der mit dem Druckübertragungselement in Kontakt ist. Insbesondere das Druckübertragungselement ist dafür ausgebildet, mit dem Infusionsschlauch für eine Erfassung einer radialen Schlauchausdehnung infolge eines aktuellen Schlauchinnendrucks in Anlage zu kommen und eine möglichst präzise und sensible Druckübertragung vom Infusionsschlauch, auf den im Drucksensorgehäuse befindlichen Druckkraftsensoren zu erlauben, um den Innendruck des Infusionsschlauchs zu messen.

Als möglicherweise geeignetes Medium zur Drückübertragung hat sich Gel empfohlen, welches in einer Kammer eines Sensorgehäuses eingesetzt/eingefüllt wird und einen aus dem Sensorgehäuse vorragenden Anlageabschnitt aufweisen kann, an welchem das Gel an einem Schlauch anliegt. Das Gel/Gelelement/Gelpad/Gelkissen bzw. der Gelkörper bildet damit ein Druckübertragungselement aus. Allerdings werden in einem Anwendungsgebiet mit aktiven medizinischen Geräten wie Infusionspumpen und/oder Dialysegeräten besonders hohe Anforderungen an die Genauigkeit der überwachten Parameter, wie insbesondere Innendrücke in Infusionsschläuchen, gestellt.

Gerade Temperaturschwankungen und die damit einhergehenden Änderungen in den Abmaßen und Materialzuständen einzelner Funktionselemente, insbesondere der gelbasierten Druckübertragungselemente, können jedoch zu großen Messschwankungen der Drucksensorik führen. In anderen Worten ausgedrückt führen Temperaturschwankungen nachweislich zu Messschwankungen, wobei bisher nicht klar war, welche material- oder geometrietechnischen Parameter durch die genannten Temperaturschwankungen beeinflusst werden, die für die Messschwankungen maßgeblich verantwortlich sind.

Der Stand der Technik bietet bisher keine gelgekoppelten Drucksensoreinrichtungen, welche die hohen Anforderungen von Infusionspumpen und Dialysegeräten (oder weiterer medizinischer Applikationen zur Blut- oder Fluiddruckmessung in einem Schlauch) stabil erfüllen und robust gegen temperaturabhängige Störeinflüsse sind. Der bisherige Stand der Technik hat somit den Nachteil, dass durch Temperatureinflüsse verursachte Messschwankungen nicht ausreichend kompensiert werden können, um zuverlässige und hochpräzise Messungen mittels gelgekoppelter Drucksensoreinrichtungen durchführen zu können.

### Zusammenfassung der Erfindung

Dementsprechend ist es das Ziel der vorliegenden Offenbarung, ein System, wie in Anspruch 1 definiert, bestehend aus einem Fluiddruckschlauch und einer Drucksensoreinrichtung bereitzustellen, das mittels gelbasierter Kopplung vom Druckübertragungselement, vorzugsweise einem Gelkörper , mit dem Fluiddruckschlauch/Infusionsschlauch verbunden ist oder verbunden werden kann und über einen möglichst breiten Temperaturbereich kontinuierlich präzise Messungen durchführen kann.

Auf der Suche nach der Lösung der technischen Aufgabe hat sich in empirischen Versuchen herausgestellt, dass vor allem die Kontaktseiten-Geometrie des Druckübertragungselements, auf die der Schlauch aufgelegt wird, und an der sich eine Kontaktfläche zwischen Druckübertragungselement und Schlauch ausbildet, einen großen Einfluss auf die Messgenauigkeit in der Drucksensoreinrichtung hat und dass sich die an der Kontaktseite des Druckübertragungselements ausbildende Kontaktfläche bei Temperaturveränderungen stark verändern kann.

Hierbei hat sich in weiterführenden Versuchen ferner herausgestellt, dass bestimmte Kontaktseiten-Geometrien von Druckübertragungselementen, Kontaktflächen zum Schlauch ausbilden, die sich weniger stark unter Temperatureinwirkung verändern, als die Kontaktflächen anderer Kontaktseiten-Geometrien. In anderen Worten ausgedrückt, die Kontaktseiten-Geometrie hat großen Einfluss darauf, wie stark sich die ausbildende Kontaktfläche zwischen Kontaktseite und Schlauch unter Temperaturschwankungen verändert und damit die Messschwankung entsprechend auslöst.

Es wurde geschlussfolgert, dass je weniger sich die Kontaktfläche bei Temperaturänderungen verändert, desto präziser kann der Druckkraftsensor Ausgangsspannungen entsprechend der im Schlauch tatsächlich vorherrschenden Drücke abgeben. Die in den Versuchen festgestellten Beobachtungen lassen sich mit der Formel zur Druckberechnung P = F x A plausibel begründen. Bleibt die sich zwischen Kontaktseite und Schlauch ausbildende Kontaktfläche nahezu konstant, lassen sich die Druckveränderungen über die gemessene Kraft konstant präzise ermitteln.

Die für die Erfindung durchgeführten Versuche belegten, dass die Messgenauigkeiten der Drucksensoreinrichtung insbesondere höher waren, wenn die am Schlauch anliegende Kontaktfläche der Kontaktseiten-Geometrie des Druckübertragungselements sich bei Temperaturveränderungen wenig veränderte.

Allerdings führten die bekannten Kontaktseiten-Geometrien aus dem Stand der Technik wie beispielsweise kreisrund oder in Tränenform längs zur Schlauchrichtung wie in der EP 1 269 145 B1 veröffentlicht, nicht zu einer Erzielung von zufriedenstellenden Messergebnissen einer gelbasierten Drucksensoreinrichtung bei Temperaturschwankungen.

Demensprechend bestand die konkrete technische Problematik zur Lösung der gestellten Aufgabe darin, eine Kontaktseiten-Geometrie zu schaffen, die im Zusammenspiel mit der restlichen, räumlichen Druckübertragungselement-Ausgestaltung eine Kontaktfläche zum Schlauch ausbilden kann, die unter Temperaturschwankungen möglichst konstant bleibt.

Diese technische Problematik zur Lösung der eingangs gestellten Aufgabe wird konkret aufgelöst durch ein System bestehend aus einem Fluiddruckschlauch und einer gelgekoppelten Drucksensoreinrichtung gemäß Anspruch 1. Vorteilhafte Weiterbildungen sind dabei Gegenstand der Unteransprüche.

Gemäß einem ersten Aspekt der Erfindung weist das System bestehend aus dem Fluiddruckschlauch und der Drucksensoreinrichtung ein, in einem Drucksensorgehäuse gelagertes Gel als Druckaufnahme- und Druckübertragungselement, welches an einem aus dem Drucksensorgehäuse frei vorragenden Druckeinleitungsabschnitt eine eine Einlegerichtung definierende Anlage- oder Kontaktseite für den in die Drucksensoreinrichtung eingelegten Fluiddruckschlauch hat und sich ausgehend von der Kontaktseite durch das Drucksensorgehäuse hindurch vorzugsweise unter Ausbildung einer Trichter- oder stufenweisen Einschnürungsform bis hin zu einem Druckkraftsensor erstreckt, auf. Dabei hat die Kontaktseite des Druckeinleitungsabschnitts eine sowohl zu deren Längs- wie auch zu deren Querachse symmetrische Form und die Längserstreckung der Kontaktseite parallel zur Einlegerichtung ist größer als in Querrichtung hierzu.

Eine geometrische Form mit den Proportionsverhältnissen der vorstehend genannten Kontaktseite, die in Längsrichtung des Schlauches größer ist als in Umfangsrichtung und dabei in ihrer Längs- wie auch Querachse symmetrisch ist, bietet den Vorteil, dass sie auch bei signifikanten Temperaturschwankungen der Drucksensoreinrichtungsumgebung damit einhergehende Materialzustände ausgleichen kann und dabei ihre Gesamtfläche kaum verändert. Gleichzeitig werden im Schlauch auftretende Druckveränderungen nach wie vor unverfälscht an das Druckübertragungselement und an den daran angeschlossenen Druckkraftsensor weitergegeben.

Die zur Längsachse des Schlauchs verlaufende achsensymmetrische Ausformung der Kontaktseite stellt eine gleichmäßig aufgeteilte Druckübertragung des Schlauches auf beide Hälften der Kontaktseite sicher. Die Achsensymmetrie zur Querachse der Kontaktseite trägt ebenfalls zur gleichmäßigen Druckverteilung entlang der Längsrichtung der Kontaktfläche bei und verhindert ungleichmäßig verteilte Spannungszustände die durch eine asymmetrische Flächenverteilung hervorgerufen werden können.

Ferner ist gemäß einem weiteren Aspekt der Erfindung bevorzugt vorgesehen, dass sich die Kontaktseite des Druckeinleitungsabschnitts in deren Längserstreckung hin zu ihren jeweiligen Enden verjüngt. Dadurch wird der vorstehend beschriebene Effekt einer höheren Temperaturschwankungsresistenz weiter verbessert.

Gemäß einem weiteren Aspekt der Erfindung kann die Kontaktfläche des Druckeinleitungsabschnitts in deren Längserstreckung spitz zulaufen oder die Form einer Raute aufweisen.

Weiterhin kann die Kontaktseite eine Außenlinie ohne Ecken bilden.

Eine Kontaktseite ohne Ecken bietet den Vorteil, dass Spannungszustände zwischen Schlauch und Druckübertragungselement durch die abgerundete Form der Kontaktseite gleichmäßiger verteilt werden, als dies in Eckbereichen mit spitzen bzw. kantigen Übergängen der Fall ist, sodass es durch die gleichmäßigere Spannungsverteilung auch zu weniger lokal-konzentrierten Verwindungen der Kontaktseite und damit einhergehenden Messverfälschungen kommt.

Des Weiteren kann die Kontaktseite nahezu die Form eines Rechtsecks aufweisen, wobei Übergange zwischen Schmalseite und Längsseite, bogenförmig sind.

Eine nahezu reckteckige Kontaktfläche bietet den Vorteil entlang eines Streifens möglichst viel Auflagefläche zu schaffen, gleichzeitig bieten als Kurven oder auch als Radien ausgeführte Übergangsbereiche zwischen Schmal und Längsseiten die Möglichkeit, auf scharf- bzw. spitzkantige Ecken zu verzichten, die zu lokal-konzentrierten Spannungszuständen führen und damit zu Verwindungen und Veränderungen bzw. Verfälschungen in den Messergebnissen führen können.

Weiterhin kann sich die Geometrie der Kontaktseite ausgehend von ihrer Querachse hin zu ihren Enden stetig verjüngen.

In einem weiteren Aspekt kann die Geometrie der Kontaktseite die Form einer Ellipse aufweisen, deren Haupt- oder Längsachse parallel zur Längsachse des Schlauchs verläuft.

Hierbei hat sich in Versuchen herausgestellt, dass sich speziell die Geometrie einer Ellipse als äußert robust gegenüber temperaturinduzierten Flächenveränderungen erweist, und daher besonders bevorzugt ist. Zusammenfassend und mit anderen Worten ausgedrückt, beruht die Erfindung einer ersten, besonders bevorzugten Ausführungsform auf einer elliptischen Geometrie der Geloberfläche bzw. Gelkontaktseite eines gelgekoppelten Drucksensors, welche zu einer optimierten Schlauch-Gel-Sensor-Kopplung führt, um die Umweltabhängigkeit der Leistungsparameter insbesondere infolge Temperaturschwankungen zu reduzieren.

Die Schlauch-Sensor-Kopplung ist entscheidend für die Leistungsfähigkeit des Sensors, da bestehende Sensoren z.B. Temperaturabhängigkeiten aufweisen, die auf instabile Kontaktseiten und Kontaktflächen sowie Grenzflächen zurückzuführen sind. Im Stand der Technik hingegen findet die Form der Kontaktseite und der sich ausbildenden Kontaktfläche im Hinblick auf die Flexibilität/konstante Leistung über den Temperaturbereich keine Berücksichtigung. Es gibt daher bis dato keinen gelgekoppelten Sensor, der die Anforderungen an eine hohe Leistungsstabilität in aktiven medizinischen Geräten, wie Infusionspumpen und/oder Dialysegeräten, erfüllt.

Um die Temperaturabhängigkeit der Drucksensoreinrichtung reduzieren zu können, ist nunmehr gemäß der vorliegenden Offenbarung die Identifizierung der optimalen Form bzw. Geometrie der Kontaktfläche zwischen Schlauch und Gel bzw. Geldpad unerlässlich, um eine konstante Sensorausgangsspannung aufgrund einer optimalen und stabilen Kontaktfläche unter verschiedenen Umgebungsbedingungen zu gewährleisten.

Aufgrund von Temperatureinflüssen ändert sich die Höhe des Gels einer gelgekoppelten Drucksensoreinrichtung. Durch die Veränderung der Höhe ist die Kontaktseite und Kontaktfläche zwischen Gel und Schlauch nicht konstant. Die Kontaktfläche des Schlauchs hat jedoch, wie vorstehend bereits ausgeführt wurde, einen direkten Einfluss auf den Ausgangspegel eines gelgekoppelten Druckkraftsensors. Eine in Schlauchlängsrichtung langgestreckte, (bezüglich der X- und Y-Achse) achssymmetrische Seiten-/Flächenform und insbesondere eine elliptische Form bzw. Geometrie der Gelfläche einer gelgekoppelten Drucksensoreinrichtung führt unerwartet zu einer optimierten Schlauch-Gel-Sensor-Kopplung, um Umgebungsabhängigkeiten (z.B. Temperaturdrift) der Sensorausgangsspannung zu reduzieren. Die sich daraus ergebenden geringeren Toleranzen und geringeren Ausgangsschwankungen eröffnen neue Anwendungsfelder z.B. für Infusionspumpen und Dialysegeräte für diese Art von Sensoren.

Der Schlauch liegt bei einer bevorzugten Anordnung von Schlauch zu Druckübertragungselement genau auf der Hauptachse der ellipsenförmigen Kontaktseite. Die Kopplungsfläche bzw. Kontaktfläche zwischen Druckübertragungselement und Schlauch ist und bleibt dadurch konstant - auch wenn sich das Gelmaterial im Druckübertragungselement durch Temperaturänderungen zusammenzieht oder ausdehnt.

Zur Abdichtung und als Schutzmaßnahme kann in der Drucksensoreinrichtung zusätzlich eine Silikonkappe und weitere trennende Materialien, vorzugsweise in Form von Membranen, zwischen dem Gel des Druckübertragungselements und dem Schlauch eingesetzt werden, welche infolge ihrer vergleichsweise dünnen Wandstärke bzw. Materialstärke keinen Einfluss auf die offenbarungsgemäßen Effekte hat.

In einem besonders bevorzugten Aspekt der Erfindung kann die Drucksensoreinrichtung eine elliptische Kontaktseite aufweisen, wobei der Querschnitt des Druckübertragungselements unterhalb des Druckeinleitungsabschnitts trichterförmig, vorzugsweise monoton abnehmend in Richtung des Druckkraftsensors zuläuft.

Gerade in Kombination ergänzen sich die elliptische Kontaktseite und das Druckübertragungselement mit trichterförmigen Querschnitt. Denn die elliptische Kontaktseite behält stabil ihre zum Schlauch ausbildende Kontaktfläche unter Temperaturveränderungen bei, während der trichterförmige Querschnitt aufgrund von verringertem Gelvolumen im Druckübertragungselement weniger durch Temperaturschwankungen beeinflusst wird und gleichzeitig die aus der Kontaktseite eingeleiteten Kräfte an den Druckkraftsensor konzentriert auf einen kleineren Querschnitt weiterleitet.

Durch die Kombination dieser beiden konstruktiven Merkmale, nämlich der elliptischen Kontaktseite und dem trichterförmigen Querschnitt wird zum einen die Messzuverlässigkeit als auch zum anderen die Messgenauigkeit und Messsensibilität des Drucksensors erhöht. Die Effekte der beiden konstruktiven Merkmale wirken somit synergetisch aufeinander ein und reduzieren insgesamt die Temperaturabhängigkeit der Drucksensoreinrichtung.

Die vorliegende Offenbarung wird nachfolgend anhand bevorzugter Ausführungsformen unter Bezugnahme auf die begleitenden Figuren näher erläutert.

### Kurzbeschreibung der Figuren

Fig. 1 ist eine Draufsicht auf eine Drucksensoreinheit 1 mit einer aus dem Stand der Technik bekannten Kontaktseiten-Geometrie,
Fig. 2 ist eine perspektivische Darstellung der Drucksensoreinrichtung gemäß einer ersten Ausführungsform der vorliegenden Offenbarung,
Fig. 3 ist eine perspektivische Darstellung der Drucksensoreinrichtung gemäß der ersten Ausführform mit Detailansicht des Druckübertragungselements,
Fig. 4 ist eine Teilschnittansicht der Drucksensoreinrichtung gemäß der ersten Ausführungsform mit einem kissenartigen Druckeinleitungsabschnitt des Druckübertragungselements, das sich monoton trichterförmig zum Druckkraftsensor verjüngt,
Fig. 5 ist eine Teilschnittansicht einer zweiten Ausführungsform mit einem kissenartigen Druckeinleitungsabschnitt des Druckübertragungselements, wobei das Druckübertragungselement unterhalb des Druckeinleitungsabschnitt zylinderförmig zum Druckkraftsensor läuft,
Fig. 6 ist eine Draufsicht auf den Auflagekörper und auf die Kontaktseite und Kontaktfläche des Druckübertragungselements der Drucksensoreinrichtung,
Fig. 7 ist eine Teilschnittansicht der Drucksensoreinrichtung gemäß einer dritten Ausführungsform mit einem Druckübertragungselement mit stufenweiser Einschnürungsform,
Fig. 8 ist eine perspektivische Darstellung der Drucksensoreinrichtung gemäß einer vierten Ausführungsform,
Fig. 9 ist eine perspektivische Darstellung des Schlauchs der Drucksensoreinrichtung mit hervorgehobener Kontaktseite,
Fig. 10 ist eine Schnittansicht senkrecht zur Längsachse des Schlauchs der Drucksensoreinrichtung gemäß der vierten Ausführungsform.

### Beschreibung der Ausführungsbeispiele

Nachstehend werden Ausführungsbeispiele der vorliegenden Offenbarung auf der Basis der zugehörigen Figuren beschrieben. Die gleichen Elemente sind mit denselben Bezugszeichen versehen. Merkmale der einzelnen Ausführungsbeispiele können untereinander ausgetauscht werden.

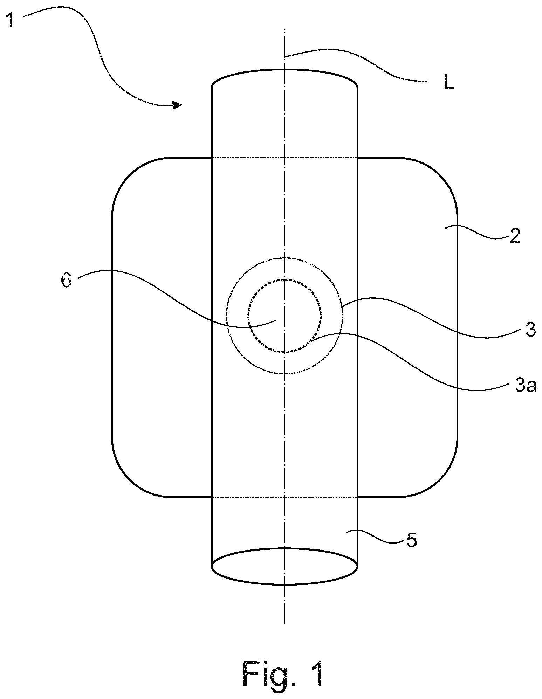

In Fig. 1 ist eine Drucksensoreinrichtung 1 mit einem Drucksensorgehäuse 2 und einer Kontaktseite 3 eines Druckübertragungselements 4 gezeigt. Auf die Kontaktseite 3 des Druckübertragungselements 4 ist ein Fluiddruckschlauch 5, im Folgenden als Schlauch bezeichnet, aufgelegt. Die Geometrie der Kontaktseite 3 entspricht hierbei bekannten Anwendungsgeometrien aus dem Stand der Technik.

Bei günstigen Temperaturbedingungen bildet sich zwischen der Kontaktseite 3 des Druckübertragungselements 4 und dem Schlauch 5 eine Kontaktfläche 3a über die gesamte Fläche der Kontaktseite 3 aus, bei Temperaturschwankungen variiert die sich zwischen Druckübertragungselement 4 und Schlauch 5 ausbildende Kontaktfläche 3a allerdings sehr stark, sodass die Kontaktfläche 3a deutlich kleiner als die Kontaktseite 3 sein kann, wie in Fig. 1 gezeigt. Durch diese ungewollten Schwankungen in der Flächenausprägung der Kontaktfläche 3a auf der Kontaktseite 3 des Druckübertragungselements kommt es infolge dessen auch zu starken Schwankungen in der Messgenauigkeit des Drucksensors 1 beim Stand der Technik.

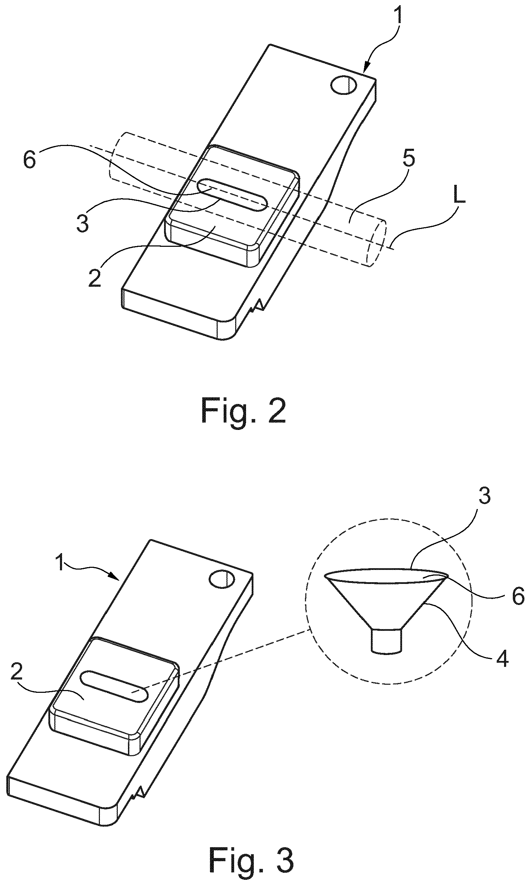

In Fig. 2 ist eine perspektivische Darstellung eines Drucksensors 1 gemäß der Erfindung gezeigt, die das zu Fig. 1 geschilderte Problem löst. Der Drucksensor 1 hat ein Drucksensorgehäuse 2 mit einer Kontaktseite 3 eines in Fig. 3 näher gezeigten Druckübertragungselements 4, auf dem ein Schlauch 5, vorzugsweise Infusionsschlauch 5, aufliegt. Besonders hervorzuheben ist hierbei die elliptische Geometrie der Kontaktseite 3, die äußert flächenstabil gegenüber Temperaturschwankungen ist, sodass die sich ausbildende Kontaktfläche 3a zwischen Druckübertragungselement 4 und Schlauch 5 konstant, nahezu der Fläche der Kontaktseite 3 entspricht.

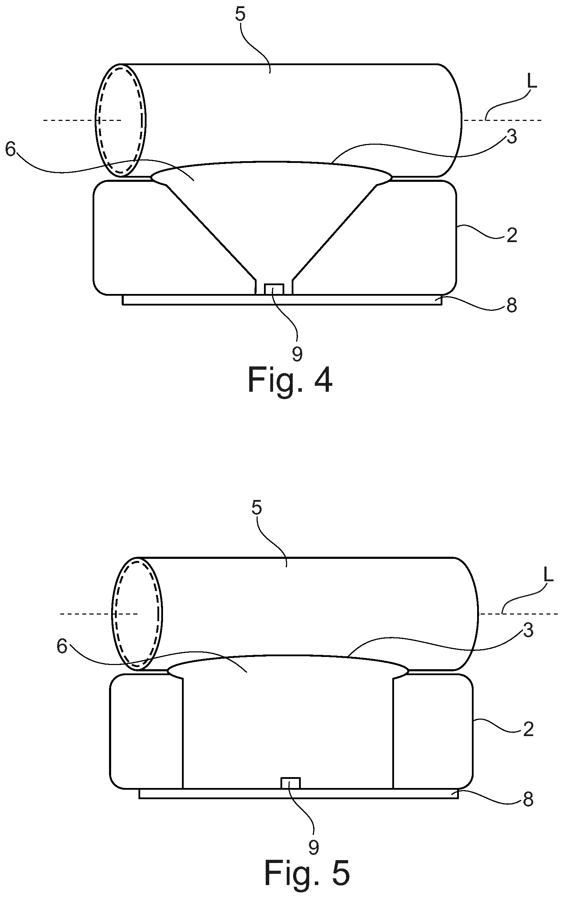

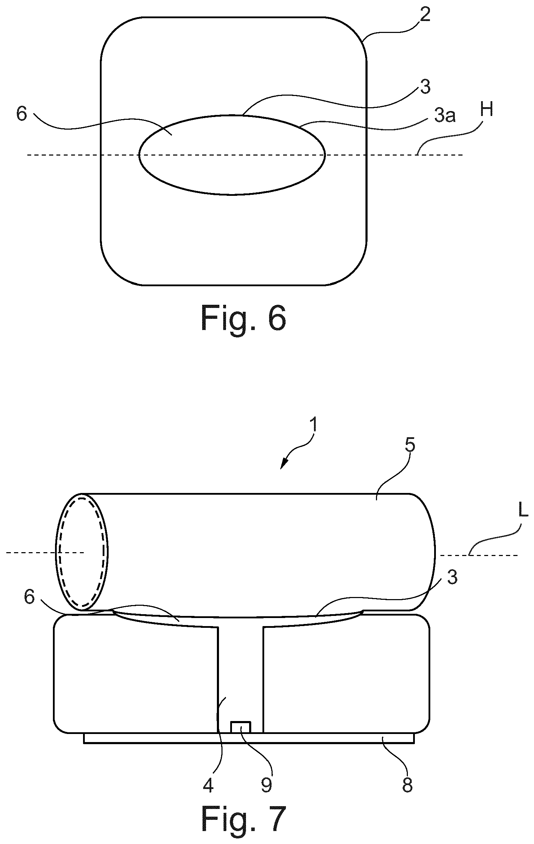

In Fig. 3 wird der Drucksensor 1 gemäß der Erfindung ohne Schlauch 5 und mit einer Detailansicht des aus dem Drucksensorgehäuse 2 herausgelösten, trichterförmigen Druckübertragungselement 4 dargestellt. Abhängig von der Ausführungsform kann die Trichterform unterschiedlich ausgeprägt sein und beispielsweise auch eine Stufenweise Einschnürungsform, wie in Fig. 7 gezeigt, aufweisen. Auch kann der zum Schlauch gelegene Druckeinleitungsabschnitt 6 des Druckübertragungselement 4 konvex oder auch konkav in Richtung des Schlauchs 5 ausgeformt sein, wie in Fig. 5 und Fig. 7 gezeigt.

In Fig. 4 wird der Drucksensor 1 der ersten Ausführungsform in einer Teilschnittansicht entlang der Längsachse L des Schlauchs 5, der selbst nicht geschnitten ist, dargestellt. Der Schlauch 5 besteht aus einem flexiblen Material, das elastisch ist und somit eine Rückstellfähigkeit hat. Der Querschnitt des Schlauchs 5 ist rund. In dem Schlauch 5 befindet sich eine Flüssigkeit, beispielweise eine Infusionslösung, die einem Patienten zugeführt wird. Wie aus Fig. 4 zu entnehmen ist, liegt der Schlauch 5 nur mittelbar auf dem Drucksensorgehäuse 2 auf und liegt unmittelbar auf einem kissenartig ausgeformten, frei vorragenden Druckeinleitungsabschnitt 6 des Druckübertragungselements 4 auf. Das Druckübertragungselement 4 erstreckt sich von einer Kontaktseite 3 am Druckeinleitungsabschnitt 6, über die der Schlauch 5 auf dem Druckübertragungselement 4 aufliegt, trichterförmig und mit elliptischer Grundfläche durch das Drucksensorgehäuse 2 hindurch und stützt sich auf einer Leiterplatte 8 sowie einem darauf angeordneten Druckkraftsensor 9 ab.

Der Druckkraftsensor 9 kann beispielsweise Dehnungsmessstreifen, die zu einer Brückenschaltung geschaltet sind, oder einen Piezo-Druckkraftsensor aufweisen. Sowohl die Dehnungsmessstreifen als auch der Piezo-Druckkraftsensor erzeugen ein elektrisches Signal, das zu der auf den Druckkraftsensor 9 einwirkenden Kraft F proportional ist.

Kommt es im Schlauch 5 durch beispielsweise Veränderungen in der Zusammensetzung des Fluids oder durch ein Verknicken des Schlauchs 5 zu Druckveränderungen, werden diese über die Kontaktseite 3 des Druckübertragungselements 4 aufgenommen und über das gelartige Kraftübertragungsmittel 4 auf den Druckkraftsensor 9 übertragen, der proportional zur einwirkenden Kraft ein elektrisches, messbares Signal abgibt.

Das Druckübertragungselement 4 besteht vorzugsweise aus inkompressiblem Gel, das sich besonders gut für die Kraftübertragung zwischen Schlauch 5 und Druckkraftsensor 9 eignet. Allerdings können sich Temperaturschwankungen auf das Volumen des Druckübertragungselements 4 signifikant auswirken, sodass sich die Höhe des Gels im Druckübertragungselement 4 verändern kann. Durch die Veränderung der Höhe ist die Kontaktseite 3 und Kontaktfläche 3a zwischen Gel und Schlauch bei bisher im Stand der Technik eingesetzten Geometrien nicht konstant.

Durch die elliptische Kopplungsfläche 3 bzw. Kontaktseite 3 gemäß der Erfindung bleibt die sich ausbildende Kontaktfläche 3a zwischen Kontaktseite 3 und Schlauch 5 konstant, auch dann, wenn sich das Gelmaterial durch Temperaturänderungen zusammenzieht oder ausdehnt. Dadurch ist es möglich, dass auch bei unterschiedlichen Temperatureinflüssen präzise Messergebnisse erzielt werden, die den hohen Standards für Infusion- und Dialyseanwendungen hinsichtlich der Messgenauigkeit, entsprechen.

Insbesondere der trichterförmige, monoton abnehmende Querschnitt des Druckübertragungselements 4 der ersten Ausführungsform des Drucksensors 1 erlaubt zum einen die Ausgestaltung einer verhältnismäßig großen Kontaktseite 3 am Druckeinleitungsabschnitt 6 hin zum Schlauch 5 und zum anderen in Richtung zum Druckkraftsensor 9 eine deutliche Querschnittsverjüngung, so dass der Abstützbereich des Druckübertragungselements 4 um den Druckkraftsensor 9 möglichst geringgehalten wird. Mit der Trichterform des Querschnitts des Druckübertragungselements 4 können somit auf einer verhältnismäßig großen Fläche Druckveränderungen im Schlauch 5 festgestellt werden und diese durch den verjüngenden Querschnitt der Trichterform möglichst konzentriert auf den Druckkraftsensor 9 übertragen werden, ohne dass ein signifikanter Teil der übertragenen Kräfte in den peripheren Bereich um den Druckkraftsensor 9 eingeleitet wird. Der trichterförmige Querschnitt des Druckübertragungselements 4 trägt somit dazu bei, dass der Druckkraftsensor 9 hochsensibel messen kann.

Ein weiterer vorteilhafter Effekt des trichterförmigen Querschnitts ist, dass Gel-Volumen im Druckübertragungselement 4, im Vergleich zu einem Druckübertragungselement 4 mit konstanten Querschnitt von Kontaktseite 3 zu Druckkraftsensor 9, eingespart werden kann. Durch die Volumenreduktion des temperaturabhängigen Gels im Druckübertragungselement 4 wird somit die Empfindlichkeit gegenüber Temperaturänderungen des Drucksensors 1 reduziert und die Messzuverlässigkeit erhöht.

Insgesamt kann gesagt werden, dass sich die positiven Effekte der elliptischen Kontaktseite 3 und des trichterförmigen Querschnitts des Druckübertragungselements 4 ergänzen. Die elliptische Kontaktseite 3 behält stabil ihre zum Schlauch 5 ausbildende Kontaktfläche 3a unter Temperaturveränderungen bei, während der trichterförmige Querschnitt aufgrund von verringerten Gelvolumen im Druckübertragungselement 4 weniger durch Temperaturschwankungen beeinflusst wird und gleichzeitig die aus der Kontaktseite 3 eingeleiteten Kräfte an den Druckkraftsensor konzentriert weiterleitet.

In Fig. 5 wird die Drucksensoreinheit 1 einer zweiten Ausführungsform in einer Teilschnittansicht entlang der Längsachse L des Schlauchs 5, der selbst nicht geschnitten ist, dargestellt. Wie aus Fig. 5 zu entnehmen ist, liegt der Schlauch 5 nur mittelbar auf dem Drucksensorgehäuse 2 auf und liegt unmittelbar auf einem kissenartig ausgeformten, frei vorragenden Druckeinleitungsabschnitt 6 des Druckübertragungselements 4 auf. Das Druckübertragungselement 4 erstreckt sich unterhalb des kissenartigen Druckeinleitungsabschnitts 6 hierbei mit konstantem zylindrischen Querschnitts mit elliptischer Grundform (von oben gesehen) bis zum Drucksensorgehäuse 2 hindurch und stützt sich auf einer Leiterplatte 8 sowie einem darauf angeordneten Druckkraftsensor 9 ab.

In Fig. 6 wird der Querschnitt der Kontaktseite 3 vor dem Hintergrund des Drucksensorgehäuses 2 in einer Draufsicht dargestellt. Die Hauptachse H der durch die Kontaktseite 3 geformten Ellipse liegt parallel zur Längsachse L (in Fig. 6 nicht gezeigt) des Schlauchs 5, wodurch sich Druckveränderungen im Schlauch 5 gleichmäßig zu beiden Seiten der Hauptachse H der Ellipse der Kontaktseite 3 verteilen. Die Kontaktseite 3 hat hierbei nahezu die identische Fläche der sich zwischen Druckübertragungselement 4 und Schlauch 5 ausbildenden Kontaktfläche 3a, wie in Fig. 6 gezeigt.

In Fig. 7 wird die Drucksensoreinheit 1 einer zweiten Ausführungsform in einer Teilschnittansicht entlang der Längsachse L des Schlauchs 5, der selbst nicht geschnitten ist, dargestellt. Das Druckübertragungselement 4 ist hierbei in einer trichterähnlichen, stufenweisen Einschnürungsform mit deutlich verringertem Gel-Volumen im Vergleich zur ersten Ausführungsform ausgeführt.

Der Druckeinleitungsabschnitt 6 des Druckübertragungsmittels 4 ist hierbei konkav in das Drucksensorgehäuse 2 gewölbt und der Querschnitt des Druckübertragungselements 4 unterhalb der Kontaktseite 3 ist schmal-zylindrisch zum Druckkraftsensor 9 ausgeformt. Die Volumenreduktion des Gels im Druckübertragungselement 4 stellt eine weitere Möglichkeit dar, den Temperatureinfluss auf das temperaturabhängige Gelvolumen des Druckübertragungselement 4 zu reduzieren und damit einhergehende Veränderungen in den Materialzuständen besser kontrollieren bzw. kompensieren zu können. Des Weiteren wird durch den vergleichsweise schmal-zylindrischen Querschnitt die Abstützfläche des Gels um den Druckkraftsensor 9 herum reduziert, wodurch die Kraftübertragung konzentrierter auf den Druckkraftsensor 9 stattfindet, sodass eine Erhöhung der Drucksensor-Sensibilität möglich ist.

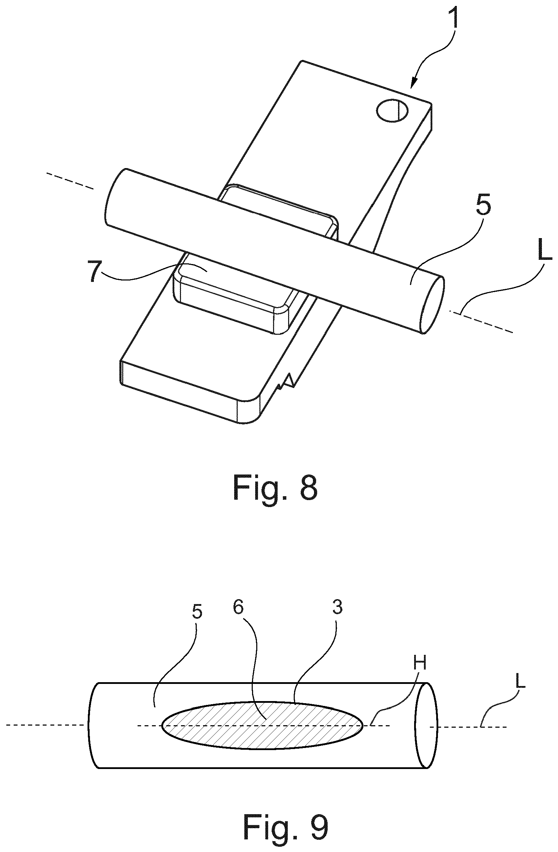

In Fig. 8 ist eine vierte Ausführungsform der Drucksensoreinrichtung 1 gezeigt, bei der das Drucksensorgehäuse 2 sowie das Druckübertragungselement 4 und der Druckkraftsensor 9 durch eine Silikonkappe 7 fluiddicht und vor Verschmutzungen geschützt sind.

Wie in Fig. 9 zu erkennen ist, wird der Schlauch 5 ebenfalls mit einer elliptischen Kontaktseite 3 auf die Silikonkappe 7 aufgelegt.

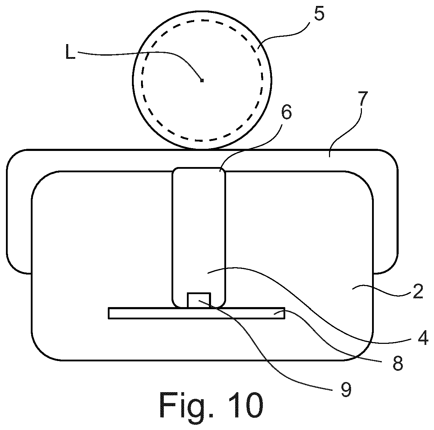

In Fig. 10 wird die Drucksensoreinrichtung 1 der vierten Ausführungsform senkrecht zur Längsachse L des Schlauchs 5, geschnitten dargestellt. Im Gegensatz zur ersten, zweiten und dritten Ausführungsform der Drucksensoreinrichtung 1, wird der Schlauch 5 in der vierten Ausführungsform nur mittelbar auf das Druckübertragungselement 4 aufgelegt und unmittelbar auf die Silikonkappe 7 aufgelegt. Die im Schlauch 5 auftretenden Druckveränderungen werden über die elastische Silikonkappe an das darunter angebundene und das sich durch das Drucksensorgehäuse 2 erstreckende Druckübertragungselement 4 übertragen und auf den auf der Leiterplatte 8 befindlichen Druckkraftsensor 9 übertragen. Die Funktionsweise der Drucksensoreinrichtung 1 der dritten Ausführungsform ist dabei identisch zur Funktionsweise der Drucksensoreinrichtung 1 der ersten und zweiten Ausführungsform, abgesehen von der zusätzlichen Abdeckung und Kraftübertragung durch die Silikonkappe 7.

## Patentansprüche

1. System bestehend aus einem Fluiddruckschlauch (5) und einer Drucksensoreinrichtung (1) mit einem, in einem Drucksensorgehäuse (2) gelagerten Gel (4) als Druckaufnahme- und Druckübertragungselement (4), welches an seinem aus dem Drucksensorgehäuse (2) frei vorragenden Druckeinleitungsabschnitt (6) eine eine Einlegerichtung definierende Anlage- oder Kontaktseite (3) für den in die Drucksensoreinrichtung (1) eingelegten Fluiddruckschlauch (5) hat und sich ausgehend von der Kontaktseite (3) durch das Drucksensorgehäuse (2) hindurch vorzugsweise unter Ausbildung einer Trichter- oder stufenweisen Einschnürungsform bis hin zu einem Druckkraftsensor (9) erstreckt, wobei die Kontaktseite (3) des Druckeinleitungsabschnitts (6) eine sowohl zu deren Längs- wie auch zu deren Querachse symmetrische Form hat,
**dadurch gekennzeichnet, dass**
die Längserstreckung der Kontaktseite (3) parallel zur Einlegerichtung größer ist als in Querrichtung hierzu.

2. System nach Anspruch 1, **dadurch gekennzeichnet, dass** sich die Kontaktseite (3) des Druckeinleitungsabschnitts (6) in deren Längserstreckung hin zu ihren jeweiligen Enden verjüngt.

3. System nach Anspruch 1 oder 2, **dadurch gekennzeichnet, dass** die Kontaktseite (3) des Druckeinleitungsabschnitts (6) in deren Längserstreckung spitz zuläuft oder die Form einer Raute aufweist.

4. System nach Anspruch 1 oder 2, **dadurch gekennzeichnet, dass** die Kontaktseite (3) eine Außenlinie ohne Ecken bildet.

5. System nach Anspruch 1 oder 2, **dadurch gekennzeichnet, dass** die Kontaktseite (3) nahezu die Form eines Rechtsecks aufweist, wobei Übergange zwischen Schmalseite und Längsseite, bogenförmig sind.

6. System nach Anspruch 1 oder 2, **dadurch gekennzeichnet, dass** sich die Geometrie der Kontaktseite (3) ausgehend von ihrer Querachse hin zu ihren Enden stetig verjüngt.

7. System nach Anspruch 1 oder 2, **dadurch gekennzeichnet, dass** die Geometrie der Kontaktseite (3) die Form einer Ellipse aufweist.

8. System nach Anspruch 7, **dadurch gekennzeichnet, dass** die Haupt- oder Längsachse (H) der Kontaktseite (3) parallel zur Längsachse (L) des einlegbaren Schlauchs (5) verläuft.

9. System nach einem der Ansprüche 1 bis 8, **gekennzeichnet, durch** eine Silikonkappe (7) und vorzugsweise weitere trennende Materialien, bevorzugt in Form von Membranen, die zwischen dem Druckübertragungselement (4) und dem Schlauch (5) zur Abdichtung und als Schutzmaßnahme angeordnet sind.

10. System nach Anspruch 7 oder 8, **dadurch gekennzeichnet, dass** sich der Querschnitt des Druckübertragungselements (4) unterhalb des Druckeinleitungsabschnitts (6) trichterförmig, vorzugsweise monoton abnehmend in Richtung des Druckkraftsensors (9) verjüngt.

## Claims

1. A system consisting of a fluid pressure hose (5) and a pressure sensor device (1) with a gel (4) mounted in a pressure sensor housing (2) as pressure absorption and pressure transmission element (4), which has, on its pressure inlet portion (6) projecting freely from the pressure sensor housing (2), an abutment or contact side (3) defining an insertion direction for the fluid pressure hose (5) inserted into the pressure sensor device (1) and, starting from the contact side (3), extends through the pressure sensor housing (2), preferably configuring a funnel or stepwise constriction shape, up to a compressive force sensor (9), wherein the contact side (3) of the pressure inlet portion (6) has a symmetrical shape both to its longitudinal axis and to its transverse axis,
**characterized in that**
the longitudinal extent of the contact side (3) parallel to the insertion direction is greater than in the transverse direction thereto.

2. The system according to claim 1, **characterized in that** the contact side (3) of the pressure inlet portion (6) tapers in its longitudinal extension towards its respective ends.

3. The system according to claim 1 or 2, **characterized in that** the contact side (3) of the pressure inlet portion (6), tapers to a point in its longitudinal extent or has the shape of a rhombus.

4. The system according to claim 1 or 2, **characterized in that** the contact side (3) forms an outer line without corners.

5. The system according to claim 1 or 2, **characterized in that** the contact side (3) has almost the shape of a rectangle, wherein transitions between narrow side and long side are arc-shaped.

6. The system according to claim 1 or 2, **characterized in that** the geometry of the contact side (3) tapers continuously from its transverse axis towards its ends.

7. The system according to claim 1 or 2, **characterized in that** the geometry of the contact side (3) has the shape of an ellipse.

8. The system according to claim 7, **characterized in that** the main or longitudinal axis (H) of the contact side (3) runs parallel to the longitudinal axis (L) of the insertable hose (5).

9. The system according to one of claims 1 to 8, **characterized by** a silicone cap (7) and preferably further separating materials, preferably in the form of membranes, which are arranged between the pressure transmission element (4) and the hose (5) for sealing and as a protective measure.

10. The system according to claim 7 or 8, **characterized in that** the cross-section of the pressure transmission element (4) below the pressure inlet portion (6) tapers in a funnel-shaped, preferably monotonously decreasing manner in the direction of the compressive force sensor (9).

## Revendications

1. Système constitué d'un tuyau de pression de fluide (5) et d'un appareil capteur de pression (1) avec un gel (4) logé dans un boîtier de capteur de pression (2) en tant qu'élément d'appui et de transmission de pression (4) qui présente, sur sa section d'introduction de pression (6) dépassant librement du boîtier de capteur de pression (2), un côté d'appui ou de contact (3) définissant une direction de mise en place pour le tuyau de pression de fluide (5) introduit dans l'appareil capteur de pression (1) et qui s'étend, en partant du côté de contact (3), à travers le boîtier de capteur de pression (2), de préférence en formant une forme d'entonnoir ou de rétrécissement par paliers, jusqu'à un capteur de force de pression (9), dans lequel le côté de contact (3) de la section d'introduction de pression (6) présente une forme symétrique à la fois par rapport à son axe longitudinal et à son axe transversal,
**caractérisé en ce que**
l'extension longitudinale du côté de contact (3) est plus grande parallèlement à la direction de mise en place que dans la direction transversale à celle-ci.

2. Système selon la revendication 1, **caractérisé en ce que** le côté de contact (3) de la section d'introduction de pression (6) se rétrécit dans son extension longitudinale vers ses extrémités respectives.

3. Système selon la revendication 1 ou 2, **caractérisé en ce que** le côté de contact (3) de la section d'introduction de pression (6) se termine en pointe dans son extension longitudinale ou présente la forme d'un losange.

4. Système selon la revendication 1 ou 2, **caractérisé en ce que** le côté de contact (3) forme une ligne extérieure sans coins.

5. Système selon la revendication 1 ou 2, **caractérisé en ce que** le côté de contact (3) présente presque la forme d'un rectangle, dans lequel des transitions entre le côté étroit et le côté longitudinal sont en forme d'arc.

6. Système selon la revendication 1 ou 2, **caractérisé en ce que** la géométrie du côté de contact (3) se rétrécit en partant de son axe transversal jusqu'à ses extrémités.

7. Système selon la revendication 1 ou 2, **caractérisé en ce que** la géométrie du côté de contact (3) présente la forme d'une ellipse.

8. Système selon la revendication 7, **caractérisé en ce que** l'axe principal ou longitudinal (H) du côté de contact (3) est parallèle à l'axe longitudinal (L) du tuyau (5) pouvant être mis en place.

9. Système selon l'une quelconque des revendications 1 à 8, **caractérisé par** un capuchon en silicone (7) et de préférence des matériaux de séparation supplémentaires, de préférence sous forme de membranes, qui sont disposés entre l'élément de transmission de pression (4) et le tuyau (5) pour l'étanchéification et comme mesure de protection.

10. Système selon la revendication 7 ou 8, **caractérisé en ce que** la section transversale de l'élément de transmission de pression (4) se rétrécit en forme d'entonnoir, de préférence de manière dégressive monotone, en direction du capteur de force de pression (9), sous la section d'introduction de pression (6).
